(19) Europäisches Patentamt
European Patent Office
Office européen des brevets

(11) Veröffentlichungsnummer: **0 164 088**
**A2**

# (12) EUROPÄISCHE PATENTANMELDUNG

(21) Anmeldenummer: **85106799.1**

(22) Anmeldetag: **03.06.85**

(51) Int. Cl.⁴: **C 07 D 249/08**, C 07 D 233/56, C 07 D 213/57, C 07 D 409/06, C 07 D 401/06, A 01 N 43/653, A 01 N 43/50, A 01 N 43/40

(30) Priorität: **07.06.84 DE 3421179**

(43) Veröffentlichungstag der Anmeldung: **11.12.85 Patentblatt 85/50**

(84) Benannte Vertragsstaaten: **AT BE CH DE FR GB IT LI NL SE**

(71) Anmelder: **BASF Aktiengesellschaft, Carl-Bosch-Strasse 38, D-6700 Ludwigshafen (DE)**

(72) Erfinder: **Roeser, Karl, Dr., Muldweg 11, D-6945 Hirschberg (DE)**
Erfinder: **Lauer, Manfred, Dr., Horst-Schork-Strasse 83, D-6700 Ludwigshafen (DE)**
Erfinder: **Sauter, Hubert, Dr., Neckarpromenade 20, D-6800 Mannheim 1 (DE)**
Erfinder: **Ammermann, Eberhard, Dr., Sachsenstrasse 3, D-6700 Ludwigshafen (DE)**
Erfinder: **Pommer, Ernst-Heinrich, Dr., Berliner Platz 7, D-6703 Limburgerhof (DE)**

(54) **Azolylnitrile und diese enthaltende Fungizide.**

(57) Verbindungen der Formel I

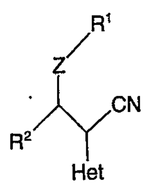

in welcher

$R_2$ für einen Heterocyclus oder für einen gegebenenfalls substituierten Phenylrest steht,

$R^1$ Alkyl, Cycloalkyl, Cycloalkylalkyl, gegebenenfalls substituiertes Aryl, gegebenenfalls substituiertes Aralkyl, gegebenenfalls substituiertes Aryloxyalkyl, gegebenenfalls substituiertes Benzyloxyalkyl, gegebenenfalls substituiertes Alkinyl oder $(CH_2-CH_2O)_m-R^4$ bedeutet, wobei $R^4$ Alkyl, gegebenenfalls substituiertes Aryl oder gegebenenfalls substituiertes Benzyl bedeutet und m die Zahlen 1, 2, 3 oder 4 bedeutet,

Het Triazolyl, Imidazolyl oder Pyridyl bedeutet,

Z eine $CH_2$-Gruppe, ein Sauerstoffatom, eine $SO_t$-Gruppe oder eine $R^3$ N-Gruppe bedeutet, wobei t die Zahlen 0, 1 oder 2 bedeutet und $R^3$ die gleichen Bedeutungen wie $R^1$ hat oder gemeinsam mit $R^1$ ein Diradikal der Formel $-(CH_2)_k-X-(CH_2)_l-$ bedeutet, wobei k und l unabhängig voneinander die Zahlen 1, 2, 3, 4 oder 5 bedeuten,

X ein O-Atom, ein S-Atom oder eine NH-Gruppe bedeutet und

Z-$R^1$ einen gegebenenfalls substituierten Phenylrest bedeutet, ihre pflanzenverträglichen Säureadditionssalze und diese enthaltende Fungizide.

EP 0 164 088 A2

## Azolylnitrile und diese enthaltende Fungizide

Die vorliegende Erfindung betrifft neue wertvolle Azolylnitrile und diese enthaltende Fungizide.

Es ist bekannt, Azolverbindungen, z.B. das 1-[2-(2,4-Dichlorphenyl)-2-(2--propenyloxy)-ethyl]-1H-imidazol, als Fungizid zu verwenden (GB-1 318 590). Die Wirkung dieser Verbindungen ist jedoch nicht befriedigend.

Es wurden neue Verbindungen der Formel I

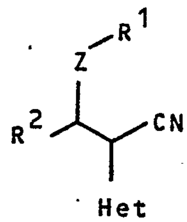

I,

gefunden, in welcher

$R^2$ für einen 5- oder 6-gliedrigen Heterocyclus mit einem oder zwei Heteroatomen oder für einen gegebenenfalls durch $R_n$ substituierten Phenylrest steht, wobei

R Wasserstoff, Alkyl, Alkoxy, Alkylthio, Alkylsulfonyl, Halogen, Halogenalkyl, Nitro, Cyano, gegebenenfalls substituiertes Phenyl oder gegebenenfalls substituiertes Phenoxy oder gegebenenfalls substituiertes Amino,

$R^1$ Alkyl, Cycloalkyl, Cycloalkylalkyl, gegebenenfalls substituiertes Aryl, gegebenenfalls substituiertes Aralkyl, gegebenenfalls substituiertes Aryloxyalkyl, gegebenenfalls substituiertes Benzyloxyalkyl, gegebenenfalls substituiertes Alkenyl, gegebenenfalls substituiertes Alkinyl oder $(CH_2-CH_2O)_m-R^4$ bedeutet, wobei $R^4$ $C_1$-$C_4$-Alkyl, gegebenenfalls substituiertes Aryl oder gegebenenfalls substituiertes Benzyl bedeutet und m die Zahlen 1, 2, 3 oder 4 bedeutet,

Het 1-(1,2,4-Triazolyl), 1-Imidazolyl oder 3-Pyridyl bedeutet,

n die Zahlen 1, 2, 3, 4 oder 5 bedeutet,

Z eine $CH_2$-Gruppe, ein Sauerstoffatom, eine $SO_t$-Gruppe oder eine $R^3$ N--Gruppe bedeutet, wobei t die Zahlen 0, 1 oder 2 bedeutet und $R^3$ die gleichen Bedeutungen wie $R^1$ hat und gleich oder verschieden von $R^1$ ist oder gemeinsam mit $R^1$ ein Diradikal der Formel $-(CH_2)_k-X-(CH_2)_l-$ bedeutet, wobei k und l unabhängig voneinander die Zahlen 1, 2, 3, 4 oder 5 bedeuten, und

X ein O-Atom, ein S-Atom oder eine NH-Gruppe bedeutet oder wobei

Z-R1 einen gegebenenfalls substituierten Phenylrest bedeutet,

Sws/P

und ihre pflanzenverträglichen Säureadditionssalze, die eine bessere fungizide Wirkung als die bekannte Verbindung haben.

In der Formel I bedeuten beispielsweise

$R^2$ einen ungesättigten heterocyclischen Rest, der z.B. 2 oder 3 Doppelbindungen im Heterocyclus enthält und 1 bis 2 Atome aus der Gruppe Sauerstoff, Schwefel und Stickstoff und insgesamt 5 bis 6 Ringglieder enthält z.B. einen Thienylrest, einen Pyridylrest, einen Furylrest, einen Pyrimidylrest oder einen Thiazolylrest, wobei vorzugsweise 2-Thienyl, 3-Thienyl, 3-Pyridyl, 4-Pyridyl, 2-Furyl geeignet sind und als Beispiel 3-Thienyl, 3-Pyridyl und 2-Furyl genannt werden.

Weiterhin steht $R^2$ für einen Phenylrest der gegebenenfalls durch $R_n$ substituiert ist. Dabei bedeutet R beispielsweise Halogen (Fluor, Chlor, Brom oder Iod), Nitro, Cyano, Alkyl mit 1 bis 4 Kohlenstoffatomen, z.B. Methyl, Ethyl, n-Propyl, iso-Propyl, Cyclopropyl, n-Butyl, iso-Butyl, sec.-Butyl, tert.-Butyl oder Cyclobutyl, $C_1$-$C_4$-Alkylsulfonyl, z.B. Methylsulfonyl, Ethylsulfonyl, n-Propylsulfonyl oder n-Butylsulfonyl, Alkoxy mit 1 bis 2 Kohlenstoffatomen (Methoxy, Ethoxy), Alkylthio mit 1 bis 2 Kohlenstoffatomen, Halogenalkyl mit 1 bis 4 Kohlenstoffatomen und 1 bis 5 Halogenatomen, insbesondere mit 1 bis 2 Kohlenstoffatomen und 1 bis 3 gleichen oder verschiedenen Halogenatomen, wobei als Halogenatome vorzugsweise Fluor oder Chlor geeignet sind und als Beispiel für Halogenalkyl Trifluormethyl genannt wird.

R kann außerdem für gegebenenfalls einfach oder mehrfach (zwei- bis dreifach), gleichartig oder verschieden substituiertes Phenyl oder Phenoxy stehen, wobei jeweils als Substituenten bevorzugt sind: Halogen, insbesondere Fluor, Chlor oder Brom; Cyano, Nitro sowie Halogenalkyl mit 1 bis 2 Kohlenstoffatomen und 1 bis 3 gleichen oder verschiedenen Halogenatomen, wobei als Halogenatome vorzugsweise Fluor oder Chlor geeignet sind und als Beispiel für Halogenalkyl Trifluormethyl genannt wird.

Gegebenenfalls substituiertes Amino ist z.B. Amino, $C_1$-$C_4$-Alkylamino, $C_1$-$C_4$-Dialkylamino.

$R^1$ steht beispielsweise für einen Alkylrest mit 1 bis 10 Kohlenstoffatomen, beispielsweise Methyl, Ethyl, n-Propyl, i-Propyl, sec.-Butyl, neo-Pentyl, n-Hexyl, n-Heptyl, n-Octyl, n-Nonyl und n-Decyl. $R^1$ kann außerdem für $C_3$-$C_8$-Cycloalkylreste stehen, z.B. Cyclopropyl, Cyclobutyl, Cyclopentyl, Cyclohexyl, Cycloheptyl, Cyclooctyl, wobei insbesondere Cyclopropyl, Cyclopentyl und Cyclohexyl genannt seien.

Ebenfalls vorteilhafte Verbindungen sind solche, in denen $R^1$ für Cycloalkyl-Alkylreste mit insgesamt 4 bis 11 Kohlenstoffatomen, z.B. für die Cyclopropylmethyl-, Cyclobutylmethyl-, Cyclopentylmethyl-, Cyclohexylmethyl-, Cycloheptylmethyl-, Cyclopropylethyl-, Cyclobutylethyl-, Cyclopentylethyl- oder Cyclohexylethyl-Gruppe steht.

Außerdem kann $R^1$ einen gegebenenfalls substituierten Phenylrest oder einen gegebenenfalls substituierten Phenylalkylrest (z.B. Benzyl) bedeuten, wobei die Substituenten Halogen (hier vorzugsweise Fluor oder Chlor), Cyano, Nitro, $C_1$- bis $C_4$-Alkyl, $C_1$-$C_4$-Alkyloxy, $C_1$-$C_4$-Alkylthioreste, Halogenalkylreste mit 1 bis 4 C-Atomen und 1 bis 9 Halogenatomen sein können. Als Beispiele seien die Benzyl-, die 4-Chlorbenzyl- und die 2,4-Dichlorbenzylgruppe genannt.

Weiterhin steht $R^1$ beispielsweise für einen gegebenenfalls substituierten Phenoxyalkylrest oder einen gegebenenfalls substituierten Benzyloxyalkylrest mit 2 bis 10 Kohlenstoffatomen in der Alkylkette und bis zu 5 gleichen oder verschiedenen Substituenten am Phenylring, die Halogen (hier vorzugsweise Fluor oder Chlor), Cyano, Nitro, $C_1$-$C_4$-Alkyl, $C_1$-$C_4$-Alkyloxy, $C_1$-$C_4$-Alkylthioreste sowie Halogenalkylreste mit 1 bis 4 C-Atomen und 1 bis 9 Halogenatomen sein können.

Als Beispiele seien Phenoxyethyl, Phenoxypropyl, Phenoxybutyl, Phenoxypentyl, Phenoxyhexyl, Phenoxyheptyl, Phenoxyoctyl, Phenoxynonyl, Phenoxydecyl, p-Chlorphenoxyethyl, p-Chlorphenoxypropyl, p-Chlorphenoxybutyl, 2,4-Dichlorphenoxyethyl, 2,4-Dichlorphenoxypropyl, 2,4-Dichlorphenoxybutyl, Benzyloxyethyl, Benzyloxypropyl, Benzyloxybutyl, Benzyloxypentyl, Benzyloxyhexyl, Benzyloxyheptyl, Benzyloxyoctyl, Benzyloxynonyl und Benzyloxydecyl genannt. Ein Alkenylrest ist z.B. ein $C_2$-$C_4$-Alkenylrest (Allyl). Ein Alkinylrest ist z.B. Propargyl oder Butinyl.

Außerdem kann $R^1$ für Reste der Formel $-(CH_2CH_2O)_m-R^4$ stehen, in denen m ganze Zahlen von 1 bis 4 bedeutet und $R^4$ einen Alkylrest mit 1 bis 4 C--Atomen, einen gegebenenfalls substituierten Phenylrest oder einen gegebenenfalls substituierten Benzylrest bedeutet. Als Beispiele seien
$-CH_2-CH_2-O-CH_3$, $-CH_2-CH_2-O-C_3H_7$, $CH_2-CH_2-O-C_4H_9$, $CH_2-CH_2-O-CH_2-CH_2-O-CH_3$,
$CH_2-CH_2-O-CH_2-CH_2-O-C_2H_5$, $CH_2-CH_2-O-CH_2-CH_2-O-C_3H_7$,
$CH_2-CH_2-O-CH_2-CH_2-O-C_4H_9$, $CH_2-CH_2-O-C_6H_5$, $CH_2-CH_2-O-$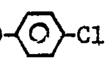$-Cl$,

$CH_2-CH_2-O-CH_2-$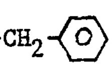, $CH_2-CH_2-O-CH_2-$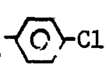$-Cl$ genannt.

Z bedeutet in der Formel I eine $CH_2$-Gruppe, ein Sauerstoffatom oder eine $SO_t$-Gruppe, wobei als Beispiele -S-, SO und SO2 genannt werden. Außerdem

kann Z für eine $R^3N$-Gruppe stehen, wobei $R^3$ aus der gleichen Gruppe von Substituenten ausgewählt ist wie $R^1$ und gleich oder verschieden von $R^1$ ist; darüber hinaus können $R^1$ und $R^3$ gemeinsam ein Diradikal der Formel $-(CH_2)_k-X-(CH_2)_l-$ bilden, wobei k und l unabhängig voneinander die Zahlen 1, 2, 3, 4 und 5 sein können und X eine $CH_2$-Gruppe, ein Sauerstoffatom, ein Schwefelatom oder eine NH-Gruppe sein kann. Beispielsweise seien für Z $CH_3-N<$, $C_2H_5-N<$, n-$C_3H_7N<$, i-$C_3H_7N<$, Phenyl-$CH_2N<$, Phenyl-$N<$

und für $-Z-R^1$ 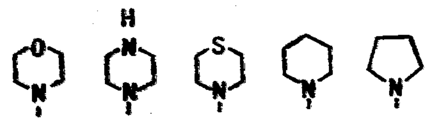 genannt.

Außerdem steht $Z-R^1$ für einen gegebenenfalls durch $R_n$ substituierten Phenylrest, in dem R und n die oben genannten Bedeutungen haben, wobei als Substituenten $R_n$ insbesondere die Halogene geeignet sind und als Beispiele 2-F-Phenyl, 3-F-Phenyl, 4-F-Phenyl, 2-Cl-Phenyl, 3-Cl-Phenyl, 4-Cl-Phenyl und 2,4-$Cl_2$-Phenyl genannt werden.

Als pflanzenverträgliche Salze der Verbindungen seien beispielsweise die Sulfate, Hydrogensulfate, Nitrate, Hydrochloride, Hydrobromide, p-Toluolsulfonate und Oxalate genannt.

M steht in Formel III für ein ein-, zwei-, drei- oder vierwertiges Metall, wobei insbesondere Li, Na, K, Mg, Ca, Al, Zr und Ti erwähnt werden und als Beispiele Li und Mg genannt seien.

Weiterhin steht M für Metallkomplexe, insbesondere für Komplexe des Kupfers. Genannt seien die Komplexe der Formel $[CuCH_2R^1]Li$, wobei $R^1$ die oben angegebene Bedeutung hat, sowie Komplexe der Formel $[CuR^5]Li$, wobei $R^5$ für einen Alkinylrest steht. Als Beispiele für solche Komplexe werden $[(CH_3)_2Cu]Li$, $[(C_2H_5)_2Cu]Li$, $[(n-C_3H_7)_2Cu]Li$, $[(n-C_4H_9)_2Cu]Li$, $[(n-C_5H_{11})_2Cu]Li$, $[(n-C_6H_{13})_2Cu]Li$, $[(n-C_7H_{15})_2Cu]Li$, $[(n-C_8H_{17})_2Cu]Li$, $[(n-C_9H_{19})_2Cu]Li$, $[(n-C_{10}H_{21})Cu]Li$, $[CH_3CuC{\equiv}C-CH_3]Li$, $[C_2H_5CuC{\equiv}C-CH_3]Li$, $[n-C_3H_7CuC{\equiv}C-CH_3]Li$, $[n-C_4H_9CuC{\equiv}C-CH_3]Li$, $[n-C_5H_{11}CuC{\equiv}C-CH_3]Li$, $[n-C_6H_{13}CuC{\equiv}C-CH_3]Li$ und $[n-C_7H_{15}CuC{\equiv}C-CH_3]Li$ genannt.

Der Rest einer Metallverbindung ist beispielsweise der Rest -MgCl, -CuCl, -CaCl, $-AlCl_2$.

Die Verbindungen der Formel I lassen sich herstellen, indem man einem der unten aufgeführten Schemata folgt:

Schema A

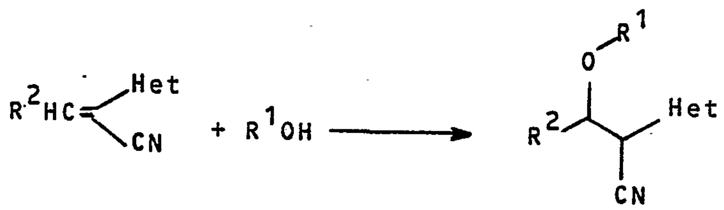

II

Schema B

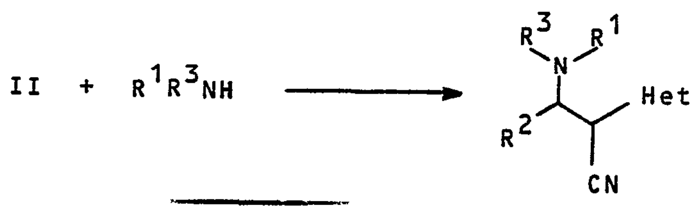

Schema C

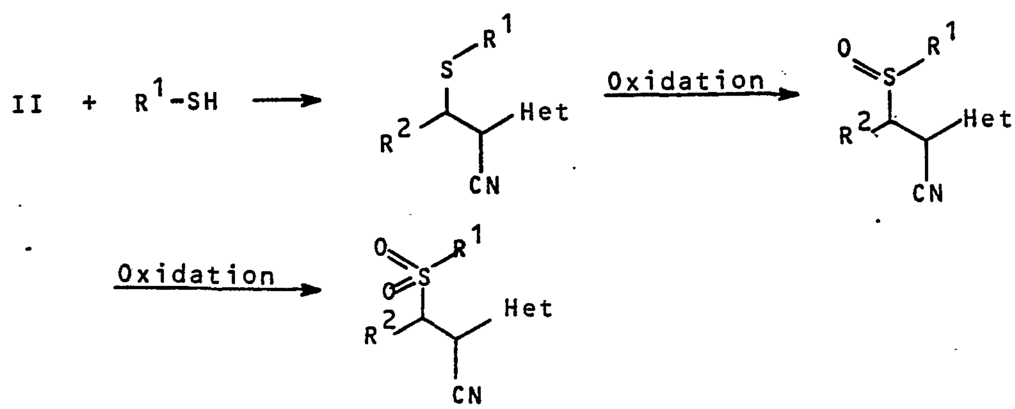


Schema D

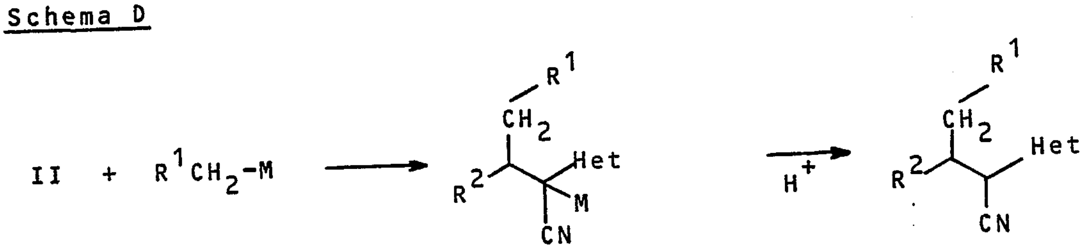


Die Reaktionen können gegebenenfalls in Gegenwart eines Verdünnungsmittels und/oder eines Katalysators durchgeführt werden. Als Verdünnungsmittel können z.B. Diethylether, Chloroform, Methylenchlorid, Toluol, Xylole, Tetrahydrofuran, Dioxan, Dimethylsulfoxid, Dimethylformamid, Methanol, Ethanol oder tert.-Butanol verwendet werden. Zweckmäßige Katalysatoren sind z.B. Amine, wie z.B. Triethylamin oder Diisopropylamin, Metallhydroxide wie Natriumhydroxid oder Kaliumhydroxid, Metallalkoholate wie z.B. Natriummethylat oder Kalium-tert.-Butylat oder Ammoniumbasen wie

Triton B. Weitere zweckmäßige Katalysatoren sind Mineralsäuren wie z.B. Schwefelsäure oder Salzsäure, organische Säuren wie z.B. Ameisensäure, Essigsäure oder p-Toluolsulfonsäure sowie saure oder basische flüssige oder feste Ionenaustauscher.

Die Zwischenprodukte der Formel II sind ebenfalls Gegenstand der Erfindung. Sie lassen sich nach an sich literaturbekannten Methoden aus den entsprechenden Aldehyden der Formel $R^2$-CHO, in der $R^2$ die oben genannten Bedeutungen hat, und Verbindungen der Struktur V, in der Het die oben angegebene Bedeutung hat, darstellen:

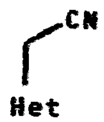 (V)

Die Herstellung der Zwischenprodukte gemäß Formel II erfolgt zweckmäßigerweise, indem man die Einsatzstoffe gegebenenfalls in Gegenwart eines Lösungs- oder Verdünnungsmittels und/oder eines Katalysators bei Temperaturen zwischen 20 und 200°C, vorzugsweise zwischen 40 und 150°C, reagieren läßt. Insbesondere lassen sich die Verbindungen herstellen, indem man das entstehende Reaktionswasser kontinuierlich oder diskontinuierlich aus dem Reaktionsgemisch entfernt, z.B. durch Auskreisen mittels eines Wasserabscheiders. Geeignete Lösungsmittel sind z.B. Diethylether, Chloroform, Methylenchlorid, Toluol, Xylol, Tetrahydrofuran, Dioxan, Dimethylformamid, Dimethylsulfoxid, Methanol, Ethanol, Isopropanol oder tert.-Butanol. Geeignete Katalysatoren sind z.B. Amine wie Triethylamin, Piperidin, Chinuclidin oder Morpholin, Metallhydroxide wie z.B. Natriumhydroxid oder Kaliumhydroxid, Metallalkoholate wie z.B. Kalium-tert.-butylat. Andere geeignete Katalysatoren sind z.B. Mineralsäuren wie z.B. Schwefelsäure, organische Säuren wie z.B. Ameisensäure, Essigsäure oder p-Toluolsulfonsäure, Salze wie Piperidiniumbenzoat oder Ammoniumacetat oder Aminosäuren wie z.B. ß-Alanin.

Je nach Reaktionsführung fallen die Zwischenprodukte der Formel II als Gemische der geometrischen Isomeren an, wobei meist eines der Isomeren dominiert. Die reinen Isomeren lassen sich daraus durch übliche Reinigungsoperationen, wie z.B. Kristallisation, Destillation oder Chromatographie, isolieren.

Zur Herstellung von erfindungsgemäßen Verbindungen der Formel I lassen sich wahlweise Isomerengemische der Formel II oder die entsprechenden reinen geometrischen Isomeren verwenden.

Die Verbindungen der Formel I enthalten mindestens zwei Chiralitätszentren und können deshalb als Enantiomeren- bzw. Diastereomerengemische vorliegen.

Die reinen Isomeren können entweder durch Isolierung aus den Isomerengemischen oder durch gezielte Synthesen erhalten werden.

Die einzelnen Isomeren haben im allgemeinen unterschiedliche biologische Wirkungen und sind als solche ebenso wie ihre Gemische Gegenstand der vorliegenden Erfindung.

Die neuen Säureadditionssalze sind durch Umsetzung von Verbindungen der allgemeinen Formel I mit Säuren erhältlich. Sie sind meist kristalline Verbindungen mit guter Pflanzenverträglichkeit.

Vorschrift 1

Herstellung des Zwischenproduktes

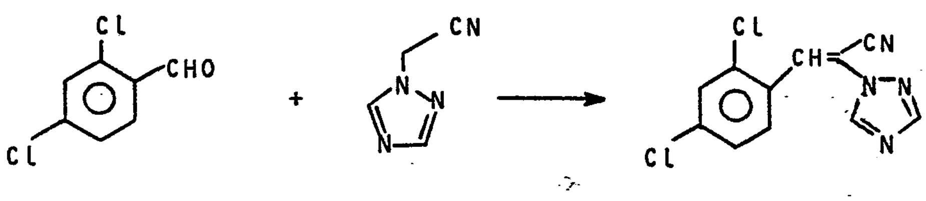

138,9 g (790 mmol) 2,4-Dichlorbenzaldehyd, 76,7 g (710 mmol) 2-(1-(1,2,4-Triazolyl)-acetonitril) und 1 g Piperidin werden in 1 l Toluol am Wasserabscheider solange zum Sieden erhitzt, bis 1 Äquivalent Wasser abgeschieden ist. Danach läßt man erkalten und saugt die ausgefallenen Kristalle ab. Zur Reinigung kann aus Toluol umkristallisiert werden. Man erhält 56,5 g (30 % d.Th.) 3-(2,4-Dichlor-phenyl)-2-(1-(1,2,4-triazolyl))-acrylnitril, Fp. 156 bis 160°C.

Auf analoge Weise lassen sich die folgenden Zwischenprodukte darstellen:

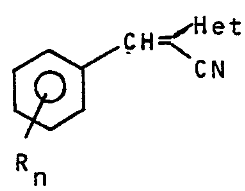

| $R_n$ | Het | Phys. Daten |
|---|---|---|
| 4-Cl | Triazol | Fp. 122-4°C |
| 2,4-$Cl_2$ | " | Fp. 156-160°C |
| 2-Cl | " | Fp. 130-1°C |

| $R_n$ | Het | Phys. Daten |
|---|---|---|
| 2-F | Triazol | Fp. 85-87°C |
| 4-F | " | Fp. 117-20°C |
| 4-$OCH_3$ | " | Fp. 132-6°C |
| 4-Phenyl | " | |
| 4-$CF_3$ | " | Fp. 63°C |
| 2-$CF_3$-4-Cl | " | |
| 2-$OCH_3$ | " | Fp. 144-147°C |
| 2,4,6-$Cl_3$ | " | |
| 2,6-$(CH_3)_2$ | " | |
| 4-$CH_3$ | " | |
| 3,5-$Cl_2$ | " | |
| 3,5-$(CH_3)_2$ | " | |
| 2-$CH_3$ | " | |
| 2,6-$Cl_2$ | " | |
| 4-$C(CH_3)_3$ | " | Fp. 65°C |
| 2,4-$Cl_2$ | Imidazol | Fp. 102°C |

Die vorliegende Erfindung umfaßt die reinen geometrischen Isomeren dieser Verbindungen ebenso wie deren Gemische.

Beispiel 1

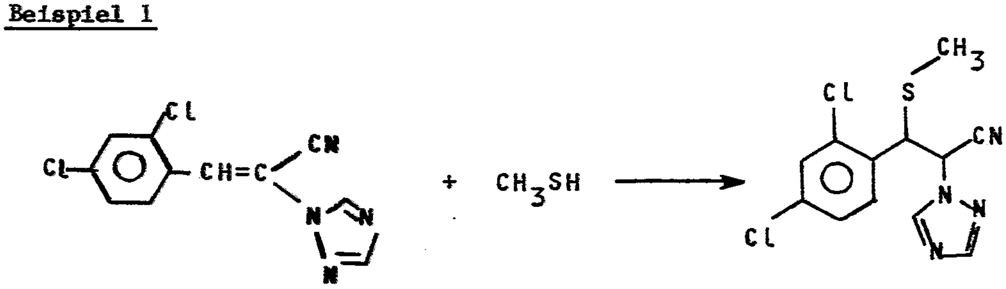

2,6 g (10 mmol) des gemäß Vorschrift 1 erhaltenen Nitrils werden in 20 ml Ethanol suspendiert. Dazu fügt man 480 mg (10 mmol) Methylmercaptan. Man rührt 14 Stunden bei Raumtemperatur und verdampft dann das Lösungsmittel unter vermindertem Druck. Der Rückstand wird an Kieselgel chromatographiert (Elutionsmittel $CH_2Cl_2$/Aceton 95:5). Man erhält so 2,8 g (91 % d.Th.) eines gelblichen Öls (Verbindung Nr. 1).

Entsprechend können durch Wahl der Ausgangsstoffe und entsprechende Anpassung der Verfahrensbedingungen die nachstehend aufgelisteten Verbindungen erhalten werden.

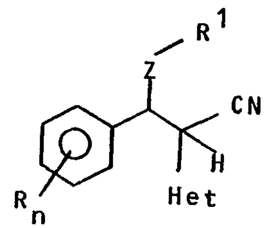

| Lfd. Nr. | $R_n$ | Het | Z | $R^1$ | IR-Daten [$cm^{-1}$] bzw. Fp [°C] |
|---|---|---|---|---|---|
| 1 | 2,4-$Cl_2$ | 1,2,4-Triazol-1-yl | S | Methyl | |
| 2 | 2,4-$Cl_2$ | 1,2,4-Triazol-1-yl | S | Ethyl | 1505,1275,1135,676 |
| 3 | 2,4-$Cl_2$ | 1,2,4-Triazol-1-yl | S | n-Propyl | 2964,1504,1474,1275 |
| 4 | 2,4-$Cl_2$ | 1,2,4-Triazol-1-yl | S | n-Butyl | |
| 5 | 2,4-$Cl_2$ | 1,2,4-Triazol-1-yl | S | n-Pentyl | 2930,1505,1275,1135,676 |
| 6 | 2,4-$Cl_2$ | 1,2,4-Triazol-1-yl | S | n-Hexyl | 2929,1505,1275,1135 |
| 7 | 2,4-$Cl_2$ | 1,2,4-Triazol-1-yl | S | n-Heptyl | 2928,1505,1275,1135 |
| 8 | 2,4-$Cl_2$ | 1,2,4-Triazol-1-yl | S | Phenyl | 1505,1474,1276,1135 |
| 9 | 2,4-$Cl_2$ | 1,2,4-Triazol-1-yl | S | 4-Cl-Benzyl | Öl |
| 10 | 4-F | 1,2,4-Triazol-1-yl | S | Methyl | |
| 11 | 4-F | 1,2,4-Triazol-1-yl | S | Ethyl | Öl |
| 12 | 4-F | 1,2,4-Triazol-1-yl | S | n-Propyl | Öl |
| 13 | 4-F | 1,2,4-Triazol-1-yl | S | n-Butyl | |
| 14 | 4-F | 1,2,4-Triazol-1-yl | S | n-Pentyl | 2930,1508,1276,1228 |
| 15 | 4-F | 1,2,4-Triazol-1-yl | S | n-Hexyl | |
| 16 | 4-F | 1,2,4-Triazol-1-yl | S | n-Heptyl | 2928,1508,1275,1218 |
| 17 | 4-F | 1,2,4-Triazol-1-yl | S | Phenyl | Öl |
| 18 | 4-F | 1,2,4-Triazol-1-yl | S | 4-Cl-Benzyl | Öl |
| 19 | 4-$OCH_3$ | 1,2,4-Triazol-1-yl | S | Methyl | |
| 20 | 4-$OCH_3$ | 1,2,4-Triazol-1-yl | S | Ethyl | |

| Lfd. Nr. | $R_n$ | Het | Z | $R^1$ | IR-Daten [$cm^{-1}$] bzw. Fp [°C] |
|---|---|---|---|---|---|
| 21 | 4-$OCH_3$ | 1,2,4-Triazol-1-yl | S | n-Propyl | 2963,1513,1257,1135 |
| 22 | 4-$OCH_3$ | 1,2,4-Triazol-1-yl | S | n-Butyl | |
| 23 | 4-$OCH_3$ | 1,2,4-Triazol-1-yl | S | n-Pentyl | 2950,1510,1755,1090 |
| 24 | 4-$OCH_3$ | 1,2,4-Triazol-1-yl | S | n-Hexyl | Fp. 98- 99 |
| 25 | 4-$OCH_3$ | 1,2,4-Triazol-1-yl | S | n-Heptyl | |
| 26 | 4-$OCH_3$ | 1,2,4-Triazol-1-yl | S | Phenyl | Fp. 125-127 |
| 27 | 4-$OCH_3$ | 1,2,4-Triazol-1-yl | S | 4-Cl-Benzyl | 2930,1560,1300,880 |
| 28 | 4-Cl | 1,2,4-Triazol-1-yl | S | Methyl | |
| 29 | 4-Cl | 1,2,4-Triazol-1-yl | S | Ethyl | |
| 30 | 4-Cl | 1,2,4-Triazol-1-yl | S | n-Propyl | 2960,1500,1270,1050 |
| 31 | 4-Cl | 1,2,4-Triazol-1-yl | S | n-Butyl | |
| 32 | 4-Cl | 1,2,4-Triazol-1-yl | S | n-Pentyl | 2950,1500,1270,1130 |
| 33 | 4-Cl | 1,2,4-Triazol-1-yl | S | n-Hexyl | 2920,1590,1500,1270 |
| 34 | 4-Cl | 1,2,4-Triazol-1-yl | S | n-Heptyl | |
| 35 | 4-Cl | 1,2,4-Triazol-1-yl | S | Phenyl | Fp. 130-131 |
| 36 | 4-Cl | 1,2,4-Triazol-1-yl | S | 4-Cl-Benzyl | |
| 37 | 2-Cl | 1,2,4-Triazol-1-yl | S | Methyl | |
| 38 | 2-Cl | 1,2,4-Triazol-1-yl | S | Ethyl | |
| 39 | 2-Cl | 1,2,4-Triazol-1-yl | S | n-Propyl | 1550,1480,1320,1180 |
| 40 | 2-Cl | 1,2,4-Triazol-1-yl | S | n-Butyl | |
| 41 | 2-Cl | 1,2,4-Triazol-1-yl | S | n-Pentyl | 2920,1590,1500,1270 |
| 42 | 2-Cl | 1,2,4-Triazol-1-yl | S | n-Hexyl | 2920,1500,1270,1180 |
| 43 | 2-Cl | 1,2,4-Triazol-1-yl | S | n-Heptyl | |
| 44 | 2-Cl | 1,2,4-Triazol-1-yl | S | Phenyl | 2960,1580,1500,1200 |
| 45 | 2-Cl | 1,2,4-Triazol-1-yl | S | 4-Cl-Benzyl | 2920,1500,1490,1090 |

| Lfd. Nr. | $R_n$ | Het | Z | $R^1$ | IR-Daten [$cm^{-1}$] bzw. Fp [°C] |
|---|---|---|---|---|---|
| 46 | 3,5-$Cl_3$ | 1,2,4-Triazol-1-yl | S | Methyl | |
| 47 | 3,5-$Cl_3$ | 1,2,4-Triazol-1-yl | S | Ethyl | |
| 48 | 3,5-$Cl_3$ | 1,2,4-Triazol-1-yl | S | n-Propyl | |
| 49 | 3,5-$Cl_3$ | 1,2,4-Triazol-1-yl | S | n-Butyl | |
| 50 | 2,4-$Cl_2$ | 1,2,4-Triazol-1-yl | O | Methyl | Fp. 128-130 |
| 51 | 2,4-$Cl_2$ | 1,2,4-Triazol-1-yl | O | Ethyl | Fp. 122-123 |
| 52 | 2,4-$Cl_2$ | 1,2,4-Triazol-1-yl | O | n-Propyl | |
| 53 | 2,4-$Cl_2$ | 1,2,4-Triazol-1-yl | O | n-Butyl | |
| 54 | 2,4-$Cl_2$ | 1,2,4-Triazol-1-yl | O | n-Pentyl | |
| 55 | 2,4-$Cl_2$ | 1,2,4-Triazol-1-yl | O | n-Hexyl | |
| 56 | 2,4-$Cl_2$ | 1,2,4-Triazol-1-yl | O | n-Heptyl | |
| 57 | 2,4-$Cl_2$ | 1,2,4-Triazol-1-yl | O | Phenyl | |
| 58 | 2,4-$Cl_2$ | 1,2,4-Triazol-1-yl | O | 4-Cl-Benzyl | |
| 59 | 2-F | 1,2,4-Triazol-1-yl | O | Methyl | |
| 60 | 2-F | 1,2,4-Triazol-1-yl | O | Ethyl | |
| 61 | 2-F | 1,2,4-Triazol-1-yl | O | n-Propyl | |
| 62 | 2-F | 1,2,4-Triazol-1-yl | O | n-Butyl | |
| 63 | 2-F | 1,2,4-Triazol-1-yl | O | n-Pentyl | |
| 64 | 2-F | 1,2,4-Triazol-1-yl | O | n-Hexyl | |
| 65 | 2-F | 1,2,4-Triazol-1-yl | O | n-Heptyl | |
| 66 | 2-F | 1,2,4-Triazol-1-yl | O | Phenyl | |
| 67 | 2-F | 1,2,4-Triazol-1-yl | O | 4-Cl-Benzyl | |
| 68 | 2-F | 1,2,4-Triazol-1-yl | $CH_2$ | Methyl | |
| 69 | 2-F | 1,2,4-Triazol-1-yl | $CH_2$ | Ethyl | |

| Lfd. Nr. | $R_n$ | Het | Z | $R^1$ | IR-Daten [$cm^{-1}$] bzw. Fp [°C] |
|---|---|---|---|---|---|
| 70 | 2-F | 1,2,4-Triazol-1-yl | $CH_2$ | n-Propyl | |
| 71 | 2-F | 1,2,4-Triazol-1-yl | $CH_2$ | n-Butyl | |
| 72 | 2-F | 1,2,4-Triazol-1-yl | $CH_2$ | n-Pentyl | |
| 73 | 2-F | 1,2,4-Triazol-1-yl | $CH_2$ | n-Hexyl | |
| 74 | 2-F | 1,2,4-Triazol-1-yl | $CH_2$ | n-Heptyl | |
| 75 | 2-F | 1,2,4-Triazol-1-yl | $CH_2$ | Phenyl | |
| 76 | 2-F | 1,2,4-Triazol-1-yl | $CH_2$ | 4-Cl-Benzyl | |
| 77 | 4-$CF_3$ | 1,2,4-Triazol-1-yl | S | Methyl | 1333,1164,1123,1073 |
| 78 | 4-$CF_3$ | 1,2,4-Triazol-1-yl | S | Ethyl | 1330,1131,1123,1071 |
| 79 | 4-$CF_3$ | 1,2,4-Triazol-1-yl | S | n-Propyl | 1328,1163,1128,1069 |
| 80 | 4-$CF_3$ | 1,2,4-Triazol-1-yl | S | n-Butyl | 1327,1163,1133,1070 |
| 81 | 4-$CF_3$ | 1,2,4-Triazol-1-yl | S | n-Pentyl | 1326,1168,1133,1071 |
| 82 | 4-$CF_3$ | 1,2,4-Triazol-1-yl | S | n-Hexyl | |
| 83 | 4-$CF_3$ | 1,2,4-Triazol-1-yl | S | n-Heptyl | |
| 84 | 4-$CF_3$ | 1,2,4-Triazol-1-yl | S | Phenyl | 1325,1168,1131,1070 |
| 85 | 4-$CF_3$ | 1,2,4-Triazol-1-yl | S | 4-Cl-Benzyl | 1328,1165,1131,1125,1069 |
| 86 | 2,4-$Cl_2$ | 1,2,4-Triazol-1-yl | $CH_2$ | Methyl | |
| 87 | 2,4-$Cl_2$ | 1,2,4-Triazol-1-yl | $CH_2$ | Ethyl | |
| 88 | 2,4-$Cl_2$ | 1,2,4-Triazol-1-yl | $CH_2$ | n-Propyl | |
| 89 | 2,4-$Cl_2$ | 1,2,4-Triazol-1-yl | $CH_2$ | n-Butyl | |
| 90 | 2,4-$Cl_2$ | 1,2,4-Triazol-1-yl | $CH_2$ | n-Pentyl | |
| 91 | 2,4-$Cl_2$ | 1,2,4-Triazol-1-yl | $CH_2$ | n-Hexyl | |
| 92 | 2,4-$Cl_2$ | 1,2,4-Triazol-1-yl | $CH_2$ | n-Heptyl | |
| 93 | 2,4-$Cl_2$ | 1,2,4-Triazol-1-yl | $CH_2$ | Phenyl | |

| Lfd. Nr. | $R_n$ | Het | Z | $R^1$ | IR-Daten [$cm^{-1}$] bzw. Fp [°C] |
|---|---|---|---|---|---|
| 94 | 2,4-$Cl_2$ | 1,2,4-Triazol-1-yl | $CH_2$ | 4-Cl-Benzyl | |
| 95 | 2-Cl | 1,2,4-Triazol-1-yl | $CH_2$ | Methyl | |
| 96 | 2-Cl | 1,2,4-Triazol-1-yl | $CH_2$ | Ethyl | |
| 97 | 2-Cl | 1,2,4-Triazol-1-yl | $CH_2$ | n-Propyl | |
| 98 | 2-Cl | 1,2,4-Triazol-1-yl | $CH_2$ | n-Butyl | |
| 99 | 2-Cl | 1,2,4-Triazol-1-yl | $CH_2$ | n-Pentyl | |
| 100 | 2-Cl | 1,2,4-Triazol-1-yl | $CH_2$ | n-Hexyl | |
| 101 | 2-Cl | 1,2,4-Triazol-1-yl | $CH_2$ | n-Heptyl | |
| 102 | 2-Cl | 1,2,4-Triazol-1-yl | $CH_2$ | Phenyl | |
| 103 | 2-Cl | 1,2,4-Triazol-1-yl | $CH_2$ | 4-Cl-Benzyl | |
| 104 | 2,4-$Cl_2$ | 1,2,4-Triazol-1-yl | SO | Methyl | |
| 105 | 2,4-$Cl_2$ | 1,2,4-Triazol-1-yl | SO | Ethyl | |
| 106 | 2,4-$Cl_2$ | 1,2,4-Triazol-1-yl | SO | n-Propyl | |
| 107 | 2,4-$Cl_2$ | 1,2,4-Triazol-1-yl | SO | n-Butyl | |
| 108 | 2,4-$Cl_2$ | 1,2,4-Triazol-1-yl | SO | n-Pentyl | |
| 109 | 2,4-$Cl_2$ | 1,2,4-Triazol-1-yl | SO | n-Hexyl | |
| 110 | 2,4-$Cl_2$ | 1,2,4-Triazol-1-yl | SO | n-Heptyl | |
| 111 | 2,4-$Cl_2$ | 1,2,4-Triazol-1-yl | SO | Phenyl | |
| 112 | 2,4-$Cl_2$ | 1,2,4-Triazol-1-yl | SO | 4-Cl-Benzyl | |
| 113 | 2,4-$Cl_2$ | 1,2,4-Triazol-1-yl | $SO_2$ | Methyl | |
| 114 | 2,4-$Cl_2$ | 1,2,4-Triazol-1-yl | $SO_2$ | Ethyl | |
| 115 | 2,4-$Cl_2$ | 1,2,4-Triazol-1-yl | $SO_2$ | n-Propyl | |
| 116 | 2,4-$Cl_2$ | 1,2,4-Triazol-1-yl | $SO_2$ | n-Butyl | |
| 117 | 2,4-$Cl_2$ | 1,2,4-Triazol-1-yl | $SO_2$ | n-Pentyl | |

| Lfd. Nr. | $R_n$ | Het | Z | $R^1$ | IR-Daten [$cm^{-1}$] bzw. Fp [°C] |
|---|---|---|---|---|---|
| 118 | 2,4-$Cl_2$ | 1,2,4-Triazol-1-yl | $SO_2$ | n-Hexyl | |
| 119 | 2,4-$Cl_2$ | 1,2,4-Triazol-1-yl | $SO_2$ | n-Heptyl | |
| 120 | 2,4-$Cl_2$ | 1,2,4-Triazol-1-yl | $SO_2$ | Phenyl | |
| 121 | 2,4-$Cl_2$ | 1,2,4-Triazol-1-yl | $SO_2$ | 4-Cl-Benzyl | |
| 122 | 2,4-$Cl_2$ | 1,2,4-Triazol-1-yl | NH | Methyl | |
| 123 | 2,4-$Cl_2$ | 1,2,4-Triazol-1-yl | NH | Ethyl | |
| 124 | 2,4-$Cl_2$ | 1,2,4-Triazol-1-yl | NH | n-Propyl | |
| 125 | 2,4-$Cl_2$ | 1,2,4-Triazol-1-yl | NH | n-Butyl | |
| 126 | 2,4-$Cl_2$ | 1,2,4-Triazol-1-yl | NH | n-Pentyl | |
| 127 | 2,4-$Cl_2$ | 1,2,4-Triazol-1-yl | NH | n-Hexyl | |
| 128 | 2,4-$Cl_2$ | 1,2,4-Triazol-1-yl | NH | n-Heptyl | |
| 129 | 2,4-$Cl_2$ | 1,2,4-Triazol-1-yl | NH | Phenyl | |
| 130 | 2,4-$Cl_2$ | 1,2,4-Triazol-1-yl | NH | 4-Cl-Benzyl | |
| 131 | 2,4-$Cl_2$ | 1,2,4-Triazol-1-yl | $NCH_3$ | Methyl | |
| 132 | 2,4-$Cl_2$ | 1,2,4-Triazol-1-yl | $NCH_3$ | Ethyl | |
| 133 | 2,4-$Cl_2$ | 1,2,4-Triazol-1-yl | $NCH_3$ | n-Propyl | |
| 134 | 2,4-$Cl_2$ | 1,2,4-Triazol-1-yl | $NCH_3$ | n-Butyl | |
| 135 | 2,4-$Cl_2$ | 1,2,4-Triazol-1-yl | $NCH_3$ | n-Pentyl | |
| 136 | 2,4-$Cl_2$ | 1,2,4-Triazol-1-yl | $NCH_3$ | n-Hexyl | |
| 137 | 2,4-$Cl_2$ | 1,2,4-Triazol-1-yl | $NCH_3$ | n-Heptyl | |
| 138 | 2,4-$Cl_2$ | 1,2,4-Triazol-1-yl | $NCH_3$ | Phenyl | |
| 139 | 2,4-$Cl_2$ | 1,2,4-Triazol-1-yl | $NCH_3$ | 4-Cl-Benzyl | |
| 140 | 2,4-$Cl_2$ | Imidazol-1-yl | S | Methyl | |
| 141 | 2,4-$Cl_2$ | Imidazol-1-yl | S | Ethyl | |

| Lfd. Nr. | $R_n$ | Het | Z | $R^1$ | IR-Daten [$cm^{-1}$] bzw. Fp [°C] |
|---|---|---|---|---|---|
| 142 | 2,4-$Cl_2$ | Imidazol-1-yl | S | n-Propyl | |
| 143 | 2,4-$Cl_2$ | Imidazol-1-yl | S | n-Butyl | |
| 144 | 2,4-$Cl_2$ | Imidazol-1-yl | S | n-Pentyl | |
| 145 | 2,4-$Cl_2$ | Imidazol-1-yl | S | n-Hexyl | |
| 146 | 2,4-$Cl_2$ | Imidazol-1-yl | S | n-Heptyl | |
| 147 | 2,4-$Cl_2$ | Imidazol-1-yl | S | Phenyl | |
| 148 | 2,4-$Cl_2$ | Imidazol-1-yl | S | 4-Cl-Benzyl | |
| 149 | 2,4-$Cl_2$ | 3-Pyridyl | S | Methyl | |
| 150 | 2,4-$Cl_2$ | 3-Pyridyl | S | Ethyl | 2930,1505,1475,1275 |
| 151 | 2,4-$Cl_2$ | 3-Pyridyl | S | n-Propyl | |
| 152 | 2,4-$Cl_2$ | 3-Pyridyl | S | n-Butyl | |
| 153 | 2,4-$Cl_2$ | 3-Pyridyl | S | n-Hexyl | 2928,1587,1470,1476 |
| 154 | 2,4-$Cl_2$ | 3-Pyridyl | S | n-Heptyl | |
| 155 | 4-F | Imidazol-1-yl | S | Methyl | |
| 156 | 4-F | Imidazol-1-yl | S | Ethyl | |
| 157 | 4-F | Imidazol-1-yl | S | n-Propyl | |
| 158 | 4-F | Imidazol-1-yl | S | n-Butyl | |
| 159 | 4-F | Imidazol-1-yl | S | n-Pentyl | |
| 160 | 4-F | Imidazol-1-yl | S | n-Hexyl | |
| 161 | 4-F | Imidazol-1-yl | S | n-Heptyl | |
| 162 | 4-F | Imidazol-1-yl | S | Phenyl | |
| 163 | 4-F | Imidazol-1-yl | S | 4-Cl-Benzyl | |
| 164 | 4-Phenyl | 1,2,4-Triazol-1-yl | S | Methyl | |
| 165 | 4-Phenyl | 1,2,4-Triazol-1-yl | S | Ethyl | |

| Lfd. Nr. | $R_n$ | Het | Z | $R^1$ | IR-Daten [$cm^{-1}$] bzw. Fp [°C] |
|---|---|---|---|---|---|
| 166 | 4-Phenyl | 1,2,4-Triazol-1-yl | S | n-Propyl | |
| 167 | 4-Phenyl | 1,2,4-Triazol-1-yl | S | n-Butyl | |
| 168 | 4-Phenyl | 1,2,4-Triazol-1-yl | S | n-Pentyl | |
| 169 | 4-Phenyl | 1,2,4-Triazol-1-yl | S | n-Hexyl | |
| 170 | 4-Phenyl | 1,2,4-Triazol-1-yl | S | n-Heptyl | |
| 171 | 4-Phenyl | 1,2,4-Triazol-1-yl | S | Phenyl | |
| 172 | 4-Phenyl | 1,2,4-Triazol-1-yl | S | 4-Cl-Benzyl | |
| 173 | 2-Methoxy | 1,2,4-Triazol-1-yl | S | Methyl | |
| 174 | 2-Methoxy | 1,2,4-Triazol-1-yl | S | Ethyl | 1504,1495,1275,1250,1135 |
| 175 | 2-Methoxy | 1,2,4-Triazol-1-yl | S | n-Propyl | |
| 176 | 2-Methoxy | 1,2,4-Triazol-1-yl | S | n-Butyl | |
| 177 | 2-Methoxy | 1,2,4-Triazol-1-yl | S | n-Pentyl | |
| 178 | 2-Methoxy | 1,2,4-Triazol-1-yl | S | n-Hexyl | |
| 179 | 2-Methoxy | 1,2,4-Triazol-1-yl | S | n-Heptyl | |
| 180 | 2-Methoxy | 1,2,4-Triazol-1-yl | S | Phenyl | |
| 181 | 2-Methoxy | 1,2,4-Triazol-1-yl | S | 4-Cl-Benzyl | |
| 182 | 4-F | 1,2,4-Triazol-1-yl | | 2-F-Phenyl | |
| 183 | 4-F | Imidazol-1-yl | | 2-F-Phenyl | |
| 184 | 2-F | 1,2,4-Triazol-1-yl | | 2-F-Phenyl | |
| 185 | 2-F | Imidazol-1-yl | | 2-F-Phenyl | |
| 186 | 2-Cl | 1,2,4-Triazol-1-yl | | 2-F-Phenyl | |
| 187 | 2-Cl | Imidazol-1-yl | | 2-F-Phenyl | |
| 188 | 4-Cl | 1,2,4-Triazol-1-yl | | 2-F-Phenyl | |
| 189 | 4-Cl | Imidazol-1-yl | | 2-F-Phenyl | |

| Lfd. Nr. | $R_n$ | Het | Z | $R^1$ | IR-Daten [$cm^{-1}$] bzw. Fp [°C] |
|---|---|---|---|---|---|
| 190 | 4-F | 1,2,4-Triazol-1-yl | | 4-F-Phenyl | |
| 191 | 4-F | Imidazol-1-yl | | 4-F-Phenyl | |
| 192 | 2-Cl | 1,2,4-Triazol-1-yl | | 4-F-Phenyl | |
| 193 | 2-Cl | Imidazol-1-yl | | 4-F-Phenyl | |
| 194 | 4-Cl | 1,2,4-Triazol-1-yl | | 4-F-Phenyl | |
| 195 | 4-Cl | Imidazol-1-yl | | 4-F-Phenyl | |

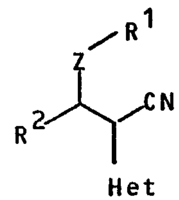

| Lfd. Nr. | $R^2$ | Het | Z | $R^1$ | IR-Daten [$cm^{-1}$] bzw. Fp [°C] |
|---|---|---|---|---|---|
| 196 | 3-Thienyl | 1,2,4-Triazol-1-yl | S | Methyl | |
| 197 | 3-Thienyl | 1,2,4-Triazol-1-yl | S | Ethyl | |
| 198 | 3-Thienyl | 1,2,4-Triazol-1-yl | S | n-Propyl | Fp. 84- 88 |
| 199 | 3-Thienyl | 1,2,4-Triazol-1-yl | S | n-Butyl | |
| 200 | 3-Thienyl | 1,2,4-Triazol-1-yl | S | n-Pentyl | 2928,1504,1433,1275 |
| 201 | 3-Thienyl | 1,2,4-Triazol-1-yl | S | n-Hexyl | 3110,2928,1504,1275 |
| 202 | 3-Thienyl | 1,2,4-Triazol-1-yl | S | n-Heptyl | |
| 203 | 3-Thienyl | 1,2,4-Triazol-1-yl | S | Phenyl | Fp. 129-132 |
| 204 | 3-Thienyl | 1,2,4-Triazol-1-yl | S | 4-Cl-Benzyl | Fp. 103-109 |

| Lfd. Nr. | $R^2$ | Het | Z | $R^1$ | IR-Daten [$cm^{-1}$] bzw. Fp [°C] |
|---|---|---|---|---|---|
| 205 | 3-Pyridyl | 1,2,4-Triazol-1-yl | S | Methyl | |
| 206 | 3-Pyridyl | 1,2,4-Triazol-1-yl | S | Ethyl | |
| 207 | 3-Pyridyl | 1,2,4-Triazol-1-yl | S | n-Propyl | 2963,1575,1504,1275 |
| 208 | 3-Pyridyl | 1,2,4-Triazol-1-yl | S | n-Butyl | |
| 209 | 3-Pyridyl | 1,2,4-Triazol-1-yl | S | n-Pentyl | 2956,1504,1426,1275 |
| 210 | 3-Pyridyl | 1,2,4-Triazol-1-yl | S | n-Hexyl | 2930,1504,1426,1275 |
| 211 | 3-Pyridyl | 1,2,4-Triazol-1-yl | S | n-Heptyl | |
| 212 | 3-Pyridyl | 1,2,4-Triazol-1-yl | S | Phenyl | Fp. 96- 99 |
| 213 | 3-Pyridyl | 1,2,4-Triazol-1-yl | S | 4-Cl-Benzyl | 2930,1504,1480,1275 |
| 214 | 4-Pyridyl | 1,2,4-Triazol-1-yl | S | Methyl | |
| 215 | 4-Pyridyl | 1,2,4-Triazol-1-yl | S | Ethyl | |
| 216 | 4-Pyridyl | 1,2,4-Triazol-1-yl | S | n-Propyl | |
| 217 | 4-Pyridyl | 1,2,4-Triazol-1-yl | S | n-Butyl | |
| 218 | 4-Pyridyl | 1,2,4-Triazol-1-yl | S | n-Pentyl | |
| 219 | 4-Pyridyl | 1,2,4-Triazol-1-yl | S | n-Hexyl | |
| 220 | 4-Pyridyl | 1,2,4-Triazol-1-yl | S | n-Heptyl | |
| 221 | 4-Pyridyl | 1,2,4-Triazol-1-yl | S | Phenyl | |
| 222 | 4-Pyridyl | 1,2,4-Triazol-1-yl | S | 4-Cl-Benzyl | |
| 223 | 2-Thienyl | 1,2,4-Triazol-1-yl | S | Methyl | |
| 224 | 2-Thienyl | 1,2,4-Triazol-1-yl | S | Ethyl | |
| 225 | 2-Thienyl | 1,2,4-Triazol-1-yl | S | n-Propyl | |
| 226 | 2-Thienyl | 1,2,4-Triazol-1-yl | S | n-Butyl | |
| 227 | 2-Thienyl | 1,2,4-Triazol-1-yl | S | n-Pentyl | |
| 228 | 2-Thienyl | 1,2,4-Triazol-1-yl | S | n-Hexyl | |
| 229 | 2-Thienyl | 1,2,4-Triazol-1-yl | S | n-Heptyl | |
| 230 | 2-Thienyl | 1,2,4-Triazol-1-yl | S | Phenyl | |
| 231 | 2-Thienyl | 1,2,4-Triazol-1-yl | S | 4-Cl-Benzyl | |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:

Erysiphe graminis (echter Mehltau) in Getreide,
Erysiphe cichoracearum an Kürbisgewächsen,
Podosphaera leucotricha an Äpfeln,
Uncinula necator an Reben,
Puccinia-Arten an Getreide,
Rhizoctonia solani an Baumwolle,
Ustilago-Arten an Getreide und Zuckerrohr,
Venturia inaequalis (Schorf) an Äpfeln,
Septoria nodorum an Weizen,
Botrytis cinerea (Grauschimmel) an Erdbeeren, Reben,
Cercospora musae an Bananen,
Pseudocercosporella herpotrichoides an Weizen, Gerste,
Pyricularia oryzae an Reis,
Hemileia vastatrix an Kaffee,
Alternaria solani an Kartoffeln, Tomaten
Plasmopara viticola an Reben sowie Fusarium- und Verticillium-Arten an verschiedenen Pflanzen.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in

bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle, (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz u.a. zur Bekämpfung holzzerstörender Pilze wie Coniophora puteanea und Polystictus versicolor eingesetzt werden. Die neuen Wirkstoffe können auch als fungizid wirksame Bestandteile öliger Holzschutzmittel zum Schutz von Holz gegen holzverfärbende Pilze eingesetzt werden. Die Anwendung erfolgt in der Weise, daß man das Holz mit diesen Mitteln behandelt, beispielsweise tränkt oder anstreicht.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gewichtsteile der Verbindung Nr. 3 mit 10 Gewichtsteilen N-Methyl-alpha-pyrrolidon und erhält eine Lösung, die zur Anwendung in Form kleinster Tropfen geeignet ist.

II. 20 Gewichtsteile der Verbindung Nr. 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von

40 Mol Ethylenoxid an 1 Mol Ricinusöl besteht. Durch Ausgießen und feines Verteilen der Lösung in Wasser erhält man eine wäßrige Dispersion.

III. 20 Gewichtsteile der Verbindung Nr. 9 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gewichtsteile der Verbindung Nr. 17 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gewichtsteile der Verbindung Nr. 8 werden den mit 3 Gewichtsteilen des Natriumsalzes der Diisobutylnaphthalin-alpha-sulfonsäure, 10 Gewichtsteilen des Natriumsalzes einer Ligninsulfonsäure aus einer Sulfitablauge und 7 Gewichtsteilen pulverförmigem Kieselsäuregel gut vermischt und in einer Hammermühle vermahlen. Durch feines Verteilen der Mischung in Wasser erhält man eine Spritzbrühe.

VI. 3 Gewichtsteile der Verbindung Nr. 9 werden mit 97 Gewichtsteilen feinteiligem Kaolin innig vermischt. Man erhält auf diese Weise ein Stäubemittel, das 3 Gew.% des Wirkstoffs enthält.

VII. 30 Gewichtsteile der Verbindung Nr. 18 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung Nr. 50 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wäßrige Dispersion. Durch Verdünnen mit Wasser erhält man eine wäßrige Dispersion.

IX. 20 Teile der Verbindung Nr. 2 werden mit 2 Teilen Calciumsalz der Dodecylbenzolsulfonsäure, 8 Teilen Fettalkoholpolyglykolether, 2 Teilen Natriumsalz eines Phenolsulfonsäure-harnstoff-formaldehyd-Kondensates und 68 Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:

Schwefel,
Dithiocarbamate und deren Derivate, wie
Ferridimethyldithiocarbamat
Zinkdimethyldithiocarbamat
Zinkethylenbisdithiocarbamat
Manganethylenbisdithiocarbamat
Mangan-Zink-ethylendiamin-bis-dithiocarbamat
Tetramethylthiuramdisulfide
Ammoniak-Komplex von Zink-(N,N'-ethylen-bis-dithiocarbamat)
Ammoniak-Komplex von Zink-(N,N'-propylen-bis-dithiocarbamat)
Zink-(N,N'-propylen-bis-dithiocarbamat)
N,N'-Polypropylen-bis-(thiocarbamoyl)-disulfid

Nitroderivate, wie
Dinitro-(1-methylheptyl)-phenylcrotonat
2-sec.-Butyl-4,6-dinitrophenyl-3,3-dimethylacrylat
2-sec.-Butyl-4,6-dinitrophenyl-isopropylcarbonat
5-Nitro-isophthalsäure-di-isopropylester,

heterocyclische Strukturen, wie
2-Heptadecyl-2-imidazolin-acetat
2,4-Dichlor-6-(o-chloranilino)-s-triazin
O,O-Diethyl-phthalimidophosphonothioat
5-Amino-1-(bis-(dimethylamino)-phosphinyl)-3-phenyl-1,2,4-triazol

2,3-Dicyano-1,4-dithiaanthrachinon
2-Thio-1,3-dithio-(4,5,6)-chinoxalin
1-(Butylcarbamoyl)-2-benzimidazol-carbaminsäuremethylester
2-Methoxycarbonylamino-benzimidazol
2-(Furyl-(2)-benzimidazol
2-(Thiazolyl-(4)-benzimidazol
N-(1,1,2,2-Tetrachlorethylthio)-tetrahydrophthalimid
N-Trichlormethylthio-tetrahydrophthalimid
N-Trichlormethylthio-phthalimid

N-Dichlorfluormethylthio-N',N'-dimethyl-N-phenyl-schwefelsäurediamid
5-Ethoxy-3-trichlormethyl-1,2,3-thiadiazol
2-Rhodanmethylthiobenzthiazol
1,4-Dichlor-2,5-dimethoxybenzol
4-(2-Chlorphenylhydrazono)-3-methyl-5-isoxazolon
Pyridin-2-thio-1-oxid
8-Hydroxychinolin bzw. dessen Kupfersalz
2,3-Dihydro-5-carboxanilido-6-methyl-1,4-oxathiin
2,3-Dihydro-5-carboxanilido-6-methyl-1,4-oxathiin-4,4-dioxid
2-Methyl-5,6-dihydro-4-H-pyran-3-carbonsäure-anilid
2-Methyl-furan-3-carbonsäureanilid
2,5-Dimethyl-furan-3-carbonsäureanilid
2,4,5-Trimethyl-furan-3-carbonsäureanilid
2,5-Dimethyl-furan-3-carbonsäurecyclohexylamid
N-Cyclohexyl-N-methoxy-2,5-dimethyl-furan-3-carbonsäureamid
2-Methyl-benzoesäure-anilid
2-Iod-benzoesäure-anilid
N-Formyl-N-morpholin-2,2,2-trichlorethylacetal
Piperazin-1,4-diylbis-(1-(2,2,2-trichlor-ethyl)-formamid
1-(3,4-Dichloranilino)-1-formylamino-2,2,2-trichlorethan
2,6-Dimethyl-N-tridecyl-morpholin bzw. dessen Salze
2,6-Dimethyl-N-cyclododecyl-morpholin bzw. dessen Salze
N-[3-(p-tert.-Butylphenyl)-2-methylpropyl]-cis-2,6-dimethylmorpholin
N-[3-(p-tert.-Butylphenyl)-2-methylpropyl]-piperidin
1-[2-(2,4-Dichlorphenyl)-4-ethyl-1,3-dioxolan-2-yl-ethyl]-1H-1,2,4-triazol
1[2-(2,4-Dichlorphenyl)-4-n-propyl-1,3-dioxolan-2-yl-ethyl]-1-H-1,2,4-
-triazol
N-(n-Propyl)-N-(2,4,6-trichlorphenoxyethyl)-N'-imidazol-yl-harnstoff
1-(4-Chlorphenoxy)-3,3-dimethyl-1-(1H-1,2,4-triazol-1-yl)-2-butanon
1-(4-Chlorphenoxy)-3,3-dimethyl-1-(1H-1,2,4-triazol-1-yl)-2-butanol
alpha-(2-Chlorphenyl)-alpha-(4-chlorphenyl)-5-pyrimidin-methanol
5-Butyl-2-dimethylamino-4-hyroxy-6-methyl-pyrimidin

Bis-(p-chlorphenyl)-3-pyridinmethanol
1,2-Bis-(3-ethoxycarbonyl-2-thioureido)-benzol
1,2-Bis-(3-methoxycarbonyl-2-thioureido)-benzol

sowie verschiedene Fungizide, wie
Dodecylguanidinacetat
3-(3-(3,5-Dimethyl-2-oxycyclohexyl)-2-hydroxyethyl)-gluataramid
Hexachlorbenzol
DL-Methyl-N-(2,6-dimethyl-phenyl)-N-furoyl(2)-alaninat,
DL-N-(2,6-Dimethyl-phenyl)-N-(2'-methoxyacetyl)-alaninmethylester
N-(2,6-Dimethylphenyl)-N-chloracetyl-D,L-2-aminobutyrolacton
DL-N-(2,6-Dimethylphenyl)-N-(phenylacetyl)-alaninmethylester
5-Methyl-5-vinyl-3-(3,5-dichlorphenyl)-2,4-dioxo-1,3-oxazolidin
3-(3,5-Dichlorphenyl(-5-methyl-5-methoxymethyl-1,3-oxazolidin-2,4-dion
3-(3,5-Dichlorphenyl)-1-isopropylcarbamoylhydantoin
N-(3,5-Dichlorphenyl)-1,2-dimethylcyclopropan-1,2-dicarbonsäureimid.
2-Cyano-N-(ethylaminocarbonyl)-2-methoximino)-acetamid
1-(2-(2,4-Dichlorphenyl)-pentyl)-1H-1,2,4-triazol
2,4-Difluor-alpha-(1H-1,2,4-triazolyl-1-methyl)-benzhydrylalkohol

Für die folgenden Versuche wurde als Vergleich der bekannte Wirkstoff 1-[2-(2,4-Dichlorphenyl)-2-(2-propenyloxy)-ethyl]-1H-imidazol (A) verwendet.

Versuch 1

Wirksamkeit gegen Botrytis cinerea an Paprika

Paprikasämlinge der Sorte "Neusiedler Ideal Elite" werden, nachdem sich 4 bis 5 Blätter gut entwickelt haben, mit wäßrigen Suspensionen, die 80 % Wirkstoff und 20 % Emulgiermittel in der Trockensubstanz enthalten, tropfnaß gespritzt. Nach dem Antrocknen des Spritzbelages werden die Pflanzen mit einer Konidienaufschwemmung des Pilzes Botrytis cinerea besprüht und bei 22 bis 24°C in eine Kammer mit hoher Luftfeuchtigkeit gestellt. Nach 5 Tagen hat sich die Krankheit auf den unbehandelten Kontrollpflanzen so stark entwickelt, daß die entstandenen Blattnekrosen den überwiegenden Teil der Blätter bedecken.

Das Ergebnis des Versuches zeigt, daß beispielsweise die Verbindungen 2, 3, 8 und 9 bei der Anwendung als 0,05 %ige Wirkstoffbrühe eine bessere fungizide Wirkung (z.B. 90 %) zeigen als der Wirkstoff A (z.B. 70 %).

Versuch 2

Wirksamkeit gegen Plasmopara viticola

Blätter von Topfreben der Sorte "Müller-Thurgau" werden mit wäßriger Spritzbrühe, die 80 % Wirkstoff und 20 % Emulgiermittel in der Trockensubstanz enthält, besprüht. Um die Wirkungsdauer der Wirkstoffe beurteilen zu können, werden die Pflanzen nach dem Antrocknen des Spritzbelages 10 Tage im Gewächshaus aufgestellt. Erst dann werden die Blätter mit einer Zoosporenaufschwemmung von Plasmopara viticola (Rebenperonospora) infiziert. Danach werden die Reben zunächst für 16 Stunden in einer wasserdampfgesättigten Kammer bei 24°C und anschließend für 8 Tage in einem Gewächshaus mit Temperaturen zwischen 20 und 30°C aufgestellt. Nach dieser Zeit werden die Pflanzen zur Beschleunigung des Sporangienträgerausbruchs abermals für 16 Stunden in der feuchten Kammer aufgestellt. Dann erfolgt die Beurteilung des Ausmaßes des Pilzausbruchs auf den Blattunterseiten.

Das Ergebnis des Versuches zeigt, daß beispielsweise die Verbindungen 2, 3, 8, 9, 17 und 18 bei der Anwendung als 0,05 %ige Wirkstoffbrühe eine bessere fungizide Wirkung (z.B. 97 %) zeigen als der Wirkstoff A (z.B. 70 %).

Versuch 3

Wirksamkeit gegen Weizenmehltau

Blätter von in Töpfen gewachsenen Weizensämlingen der Sorte "Jubilar" werden mit wäßriger Spritzbrühe, die 80 % Wirkstoff und 20 % Emulgiermittel in der Trockensubstanz enthält, besprüht und 24 Stunden nach dem Antrocknen des Spritzbelages mit Oidien (Sporen) des Weizenmehltaus (Erysiphe graminis var. tritici) bestäubt. Die Versuchspflanzen werden anschließend im Gewächshaus bei Temperaturen zwischen 20 und 22°C und 75 bis 80 % relativer Luftfeuchtigkeit aufgestellt. Nach 7 Tagen wird das Ausmaß der Mehltauentwicklung ermittelt.

Das Ergebnis des Versuches zeigt, daß beispielsweise der Wirkstoff 50 bei der Anwendung als 0,006 oder 0,0015 %ige Wirkstoffbrühe eine bessere fungizide Wirkung (z.B. 100 %) zeigt als der Wirkstoff A (z.B. 90 %).

Patentansprüche

1. Verbindung der Formel

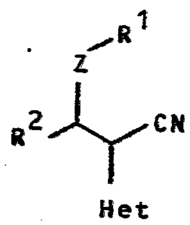

I,

in welcher

$R^2$ für einen 5- oder 6-gliedrigen Heterocyclus mit einem oder zwei Heteroatomen oder für einen gegebenenfalls durch $R_n$ substituierten Phenylrest steht, wobei

R Wasserstoff, Alkyl, Alkoxy, Alkylthio, Alkylsulfonyl, Halogen, Halogenalkyl, Nitro, Cyano, gegebenenfalls substituiertes Phenyl oder gegebenenfalls substituiertes Phenoxy oder gegebenenfalls substituiertes Amino,

$R^1$ Alkyl, Cycloalkyl, Cycloalkylalkyl, gegebenenfalls substituiertes Aryl, gegebenenfalls substituiertes Aralkyl, gegebenenfalls substituiertes Aryloxyalkyl, gegebenenfalls substituiertes Benzyloxyalkyl, gegebenenfalls substituiertes Alkenyl, gegebenenfalls substituiertes Alkinyl oder $(CH_2-CH_2O)_m-R^4$ bedeutet, wobei $R^4$ $C_1-C_4$-Alkyl, gegebenenfalls substituiertes Aryl oder gegebenenfalls substituiertes Benzyl bedeutet und m die Zahlen 1, 2, 3 oder 4 bedeutet,

Het 1-(1,2,4-Triazolyl), 1-Imidazolyl oder 3-Pyridyl bedeutet,

n die Zahlen 1, 2, 3, 4 oder 5 bedeutet,

Z eine $CH_2$-Gruppe, ein Sauerstoffatom, eine $SO_t$-Gruppe oder eine $R^3$ N-Gruppe bedeutet, wobei t die Zahlen 0, 1 oder 2 bedeutet und $R^3$ die gleichen Bedeutungen wie $R^1$ hat und gleich oder verschieden von $R^1$ ist oder gemeinsam mit $R^1$ ein Diradikal der Formel $-(CH_2)_k-X-(CH_2)_l-$ bedeutet, wobei k und l unabhängig voneinander die Zahlen 1, 2, 3, 4 oder 5 bedeuten und X ein O-Atom, ein S-Atom oder eine NH-Gruppe bedeutet oder wobei

$Z-R^1$ einen gegebenenfalls substituierten Phenylrest bedeutet

und ihre pflanzenverträglichen Säureadditionssalze.

2. Verfahren zur Herstellung von Verbindungen gemäß Anspruch 1, <u>dadurch gekennzeichnet</u>, daß man eine Verbindung der Formel II

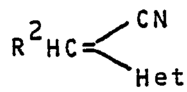 II

in der $R^2$ und Het die oben angegebene Bedeutung haben, in Form ihrer reinen geometrischen Isomeren oder als Gemische solcher Isomerer mit einer Verbindung der Formel

$R^1$ - Z - M III

umsetzt, in der $R^1$ und Z die im Anspruch 1 angegebene Bedeutung haben und M für Wasserstoff, ein Metallatom, den Rest einer Metallverbindung oder einen Metallkomplex steht.

3. Verbindung der Formel

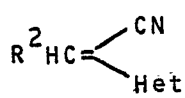 II

in der $R^2$ und Het die im Anspruch 1 genannten Bedeutungen haben, außer solchen Verbindungen, in denen Het für einen 3-Pyridylrest steht.

4. Verbindungen der Formel II gemäß Anspruch 2 mit den Strukturformeln

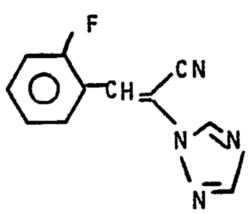

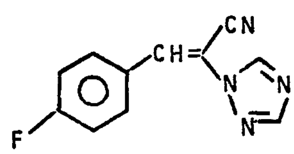

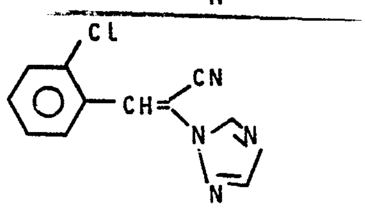

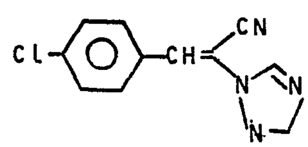

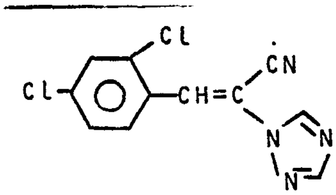

5. Verfahren zur Herstellung substituierter Acrylnitrile der Formel II,

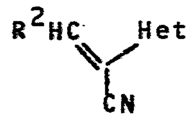

(II)

dadurch gekennzeichnet, daß man einen Aldehyd der Formel IV,

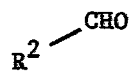

(IV)

in der $R^2$ die oben angegebene Bedeutung hat, mit einem Nitril der Formel V,

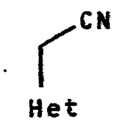

(V)

in der Het die oben angegebene Bedeutung hat, gegebenenfalls in Gegenwart eines Verdünnungsmittels und/oder eines Katalysators umsetzt.

6. Fungizid, enthaltend eine Verbindung gemäß Anspruch 1.

7. Fungizid, enthaltend einen festen oder flüssigen Trägerstoff und eine Verbindung gemäß Anspruch 1.

8. Verfahren zur Herstellung eines Fungizids, dadurch gekennzeichnet, daß man einen festen oder flüssigen Trägerstoff vermischt mit einer Verbindung gemäß Anspruch 1.

9. Verfahren zur Bekämpfung von Pilzen, dadurch gekennzeichnet, daß man die Pilze oder die vor Pilzbefall zu schützenden Materialien, Pflanzen, Boden oder Saatgüter behandelt mit einer Verbindung gemäß Anspruch 1.

10. Verbindung gemäß Anspruch 1, in der $R^2$ Halophenyl, Het Triazol, Z Schwefel oder Sauerstoff und $R^1$ $C_1$–$C_4$-Alkyl, Phenyl oder Halobenzyl bedeutet.

Patentansprüche

1. Fungizid, enthaltend eine Verbindung der Formel

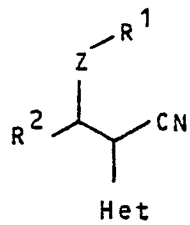

I,

in welcher

$R^2$ für einen 5- oder 6-gliedrigen Heterocyclus mit einem oder zwei Heteroatomen oder für einen gegebenenfalls durch $R_n$ substituierten Phenylrest steht, wobei

R Wasserstoff, Alkyl, Alkoxy, Alkylthio, Alkylsulfonyl, Halogen, Halogenalkyl, Nitro, Cyano, gegebenenfalls substituiertes Phenyl oder gegebenenfalls substituiertes Phenoxy oder gegebenenfalls substituiertes Amino,

$R^1$ Alkyl, Cycloalkyl, Cycloalkylalkyl, gegebenenfalls substituiertes Aryl, gegebenenfalls substituiertes Aralkyl, gegebenenfalls substituiertes Aryloxyalkyl, gegebenenfalls substituiertes Benzyloxyalkyl, gegebenenfalls substituiertes Alkenyl, gegebenenfalls substituiertes Alkinyl oder $(CH_2-CH_2O)_m-R^4$ bedeutet, wobei $R^4$ $C_1$-$C_4$-Alkyl, gegebenenfalls substituiertes Aryl oder gegebenenfalls substituiertes Benzyl bedeutet und m die Zahlen 1, 2, 3 oder 4 bedeutet,

Het 1-(1,2,4-Triazolyl), 1-Imidazolyl oder 3-Pyridyl bedeutet,

n die Zahlen 1, 2, 3, 4 oder 5 bedeutet,

Z eine $CH_2$-Gruppe, ein Sauerstoffatom, eine $SO_t$-Gruppe oder eine $R^3$ N-Gruppe bedeutet, wobei t die Zahlen 0, 1 oder 2 bedeutet und $R^3$ die gleichen Bedeutungen wie $R^1$ hat und gleich oder verschieden von $R^1$ ist oder gemeinsam mit $R^1$ ein Diradikal der Formel $-(CH_2)_k-X-(CH_2)_l-$ bedeutet, wobei k und l unabhängig voneinander die Zahlen 1, 2, 3, 4 oder 5 bedeuten und X ein O-Atom, ein S-Atom oder eine NH-Gruppe bedeutet oder wobei

$Z-R^1$ einen gegebenenfalls substituierten Phenylrest bedeutet

oder ihre pflanzenverträglichen Säureadditionssalze.

2. Verfahren zur Herstellung von Verbindungen gemäß Anspruch 1, dadurch gekennzeichnet, daß man eine Verbindung der Formel II

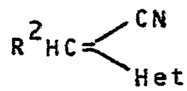 II

in der $R^2$ und Het die oben angegebene Bedeutung haben, in Form ihrer reinen geometrischen Isomeren oder als Gemische solcher Isomerer mit einer Verbindung der Formel

$$R^1 - Z - M \qquad III$$

umsetzt, in der $R^1$ und Z die im Anspruch 1 angegebene Bedeutung haben und M für Wasserstoff, ein Metallatom, den Rest einer Metallverbindung oder einen Metallkomplex steht.

3. Verfahren zur Herstellung substituierter Acrylnitrile der Formel II,

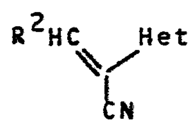 (II)

dadurch gekennzeichnet, daß man einen Aldehyd der Formel IV,

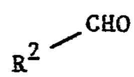 (IV)

in der $R^2$ die oben angegebene Bedeutung hat, mit einem Nitril der Formel V,

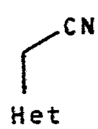 (V)

in der Het die oben angegebene Bedeutung hat, gegebenenfalls in Gegenwart eines Verdünnungsmittels und/oder eines Katalysators umsetzt.

4. Fungizid, enthaltend einen festen oder flüssigen Trägerstoff und eine Verbindung gemäß Anspruch 1.

5. Verfahren zur Herstellung eines Fungizids, dadurch gekennzeichnet, daß man einen festen oder flüssigen Trägerstoff vermischt mit einer Verbindung gemäß Anspruch 1.

6. Verfahren zur Bekämpfung von Pilzen, dadurch gekennzeichnet, daß man die Pilze oder die vor Pilzbefall zu schützenden Materialien, Pflanzen, Boden oder Saatgüter behandelt mit einer Verbindung gemäß Anspruch 1.